(19) **Europäisches Patentamt**
**European Patent Office**
**Office européen des brevets**

(11) **EP 3 467 497 B1**

(12) **EUROPEAN PATENT SPECIFICATION**

(45) Date of publication and mention
of the grant of the patent:
**29.09.2021  Bulletin 2021/39**

(51) Int Cl.:
*G01N 33/53* *(2006.01)*    *G01N 15/14* *(2006.01)*
*G01N 21/64* *(2006.01)*    *G01N 33/48* *(2006.01)*
*G01N 33/49* *(2006.01)*    *G01N 33/536* *(2006.01)*
*G01N 33/543* *(2006.01)*    *G01N 33/569* *(2006.01)*
*G01N 33/68* *(2006.01)*

(21) Application number: **17802794.2**

(22) Date of filing: **23.05.2017**

(86) International application number:
**PCT/JP2017/019165**

(87) International publication number:
**WO 2017/204208 (30.11.2017 Gazette 2017/48)**

(54) **METHOD FOR ANALYZING EXPRESSION OF SMN PROTEIN NUCLEAR BODY**

VERFAHREN ZUR ANALYSE DER EXPRESSION DES KERNKÖRPERS VON SMN-PROTEIN

PROCÉDÉ D'ANALYSE D'EXPRESSION DE CORPS NUCLÉAIRE DE LA PROTÉINE SMN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.05.2016  JP 2016103482**

(43) Date of publication of application:
**10.04.2019  Bulletin 2019/15**

(73) Proprietors:
• **Tokyo Women's Medical University
Tokyo 162-8666 (JP)**
• **Microbial Chemistry Research Foundation
Tokyo 141-0021 (JP)**

(72) Inventors:
• **SAITO, Kayoko
Tokyo 162-8666 (JP)**
• **ARAKAWA, Reiko
Tokyo 162-8666 (JP)**
• **ARAKAWA, Masayuki
Tokyo 141-0021 (JP)**

(74) Representative: **Cabinet Beau de Loménie
158, rue de l'Université
75340 Paris Cedex 07 (FR)**

(56) References cited:
**WO-A1-2015/152410    WO-A2-2006/050451
JP-A- 2012 526 046    JP-A- 2017 029 116
US-A1- 2014 349 938**

• **DIONE T. KOBAYASHI ET AL: "Evaluation of Peripheral Blood Mononuclear Cell Processing and Analysis for Survival Motor Neuron Protein", PLOS ONE, vol. 7, no. 11, 30 November 2012 (2012-11-30), page e50763, XP055389287, DOI: 10.1371/journal.pone.0050763**
• **ARAKAWA REIKO ET AL: "Imaging Flow Cytometry Analysis to Identify Differences of Survival Motor Neuron Protein Expression in Patients With Spinal Muscular Atrophy", PEDIATRIC NEUROLOGY, ELSEVIER SCIENCE, NL, vol. 61, 26 May 2016 (2016-05-26), pages 70-75, XP029679321, ISSN: 0887-8994, DOI: 10.1016/J.PEDIATRNEUROL.2016.05.009**
• **NORIKO OTSUKI ET AL: "A new biomarker candidate for spinal muscular atrophy: Identification of a peripheral blood cell population capable of monitoring the level of survival motor neuron protein", PLOS ONE, vol. 13, no. 8, 13 August 2018 (2018-08-13), page e0201764, XP55623513, DOI: 10.1371/journal.pone.0201764**
• **ARAKAWA, MASAYUKI: "A novel evaluation method of survival motor neuron protein as a biomarker of spinal muscular atrophy by imaging flow cytometry", Biochem Biophys Res Commun, vol. 453, no. 3, 24 October 2014 (2014-10-24), pages 368-374, XP029117054,**

EP 3 467 497 B1

**(Cont. next page)**

- **REIKO ARAKAWA: "Imaging Flow Cytometry-ho o Mochiita Shinki SMN Tanpakushitsu Kaisekiho", Annual Meeting of the Japan Society of Human Genetics Program Shorokushu, vol. 60, 2015, page 295,**
- **ARAKAWA REIKO: "Development of SMN protein analysis in human blood cells as a spinal muscular atrophy biomarker", Annual Meeting of the Japan Society of Human Genetics Program Shorokushu, vol. 61 6 April 2016 (2016-04-06), page 1, XP009516455, ISSN: 1434-5161 [retrieved on 2017-08-07]**
- **"Nanbyo Chiryo Kenkyu de Survival Motor Neuron no Kashika & Teiryoka ni Imaging Flow Cytometer o Shiyo", Merck Life Science Research & Biology, Retrieved from the Internet: URL:http://www.merckmillipore.com/JP/ja/20 160715_072009> [retrieved on 2017-08-07]**
- **RENUSCH, SAMANTHA R: "Spinal Muscular Atrophy Biomarker Measurements from Blood Samples in a Clinical Trial of Valproic Acid in Ambulatory Adults", J Neuromuscul Dis, vol. 2, no. 2, 4 June 2015 (2015-06-04), pages 119-130, XP055562662,**
- **CZECH , CHRISTIAN: "Biomarker for Spinal Muscular Atrophy: Expression of SMN in Peripheral Blood of SMA Patients and Healthy Controls", PLoS One, vol. 10, no. 10, 2015, page e0139950, XP055562664,**
- **PENA, E: "Neuronal body size correlates with the number of nucleoli and Cajal bodies, and with the organization of the splicing machinery in rat trigeminal ganglion neurons", J Comp Neurol, vol. 430, no. 2, 5 February 2001 (2001-02-05), pages 250-263, XP055562674,**

**Description**

Technical Field

The present invention relates to a method for analyzing the expression of a survival motor neuron (SMN) protein nuclear body.

Background Art

Spinal muscular atrophy (SMA) is a muscular atrophy caused by a lesion of anterior horn cells of the spinal cord, and shows a lower motor neuron sign characterized by muscle weakness and muscle atrophy in the trunk and limbs. SMA is classified into type I to type IV according to the age of onset and severity: type I which is severe type (also referred to as Werdnig—Hoffmann disease) and occurs by the age of six months after birth; type II which is intermediate type (also referred to as Dubowitz disease) and occurs by the age of one year and six months; and type III which is mild type (also referred to as Kugelberg-Welander disease) and occurs after the age of one year and six months. These are childhood SMA. On the other hand, type IV which occurs at the age of 20 years or older is adult SMA.

SMA type I accounts for approximately 30% of SMA and occurs at the age of six months or younger. The symptom is quite serious. The affected individual cannot keep sitting throughout life, and can rarely survive for two years or longer without artificial respiration. SMA type II makes it impossible for the affected individual to stand up and walk throughout life. The person affected with SMA type III can walk independently, but a symptom gradually appears such that the person tends to fall over, cannot walk, or cannot stand up. Despite these facts, the method for completely curing SMA has not been established yet, and SMA is one of diseases designated as intractable diseases by the country.

The responsible gene in many childhood SMA cases is the SMN1 gene located in 5q13 on a long arm of chromosome 5. In many childhood SMA cases, a deletion or mutation of the SMN1 gene has been observed, so that childhood SMA is recognized as an autosomal recessive genetic disorder. The SMN1 gene expresses SMN protein, and the SMN protein is conceivably involved in the development of spinal motor neuron and so forth.

In addition, in the same region on chromosome 5 where the SMN1 gene is located, the SMN2 gene exists which is different from the SMN1 gene by only one base in the coding region. In childhood SMA patients, the SMN1 gene is deleted or mutated, and the SMN protein derived from the SMN1 gene decreases, while only the SMN2 gene functions. Thus, the SMN protein is expressed from the SMN2 gene in childhood SMA patients, and it is believed that the severity of the symptom varies depending on the amount of the SMN2 gene-derived SMN protein expressed.

However, two types of transcription products of the SMN2 gene exist, while the transcription product of the SMN1 gene is one type of full-length SMN1 mRNA. Assuming that the amount of full-length SMN1 mRNA transcribed from the SMN1 gene is 100%, full-length SMN2 mRNA accounts for approximately 10% of the transcription products of the SMN2 gene, and truncated SMN2 mRNA in which deletion of exon 7 is observed accounts for approximately 90% of the transcription products of the SMN2 gene. The truncated SMN2 mRNA is translated into a non-functional protein, and only the full-length SMN2 mRNA is normally translated into the SMN protein. Therefore, in childhood SMA patients in which the SMN1 gene is deleted or mutated, the amount of the SMN protein expressed is low in comparison with healthy persons and is only about 10% to 20% of that in healthy persons. Hence, the deletion or mutation of the SMN1 gene causes muscle weakness and muscle atrophy.

Also in some cases of adult SMA patients, which are fewer than childhood SMA patients, the deletion of mutation of the SMN1 gene is observed. It is also believed that the severity of the symptom varies depending on the amount of the SMN protein expressed.

In SMA patients having the SMN1 gene deleted, the SMN2 gene may be present in place of the SMN1 gene. In this case, two copies of the SMN2 gene exist on one chromosome. The amount of the SMN2 gene-derived SMN protein expressed is also increased depending on the number of copies. It has been known that, in SMA patients, the larger the amount of the SMN2 gene-derived SMN protein expressed, the milder the symptom.

As described above, the amount of the SMN protein expressed is closely related to childhood SMA. It has been believed that the SMN protein can be a biomarker useful for diagnosing SMA, in particular, childhood SMA and for accurately determining the effect of an agent. However, in SMA patients also, the expression of the SMN protein per se is observed, and utilizing the SMN protein as a biomarker useful for SMA requires some sensitivity enough to detect a difference in the level of the SMN protein expressed when a patient is compared with a healthy person.

Moreover, SMA is an autosomal recessive genetic disorder, and when the SMN1 gene is deleted or mutated in only one of a pair of autosomes, the symptoms of muscle weakness and muscle atrophy do not appear at all, but the person is a carrier. In the carrier, the amount of the SMN protein expressed is larger than that in the patient. However, the difference between a carrier and a patient is presumably not always as large as the difference between a healthy person and a patient in the amount of the SMN protein expressed. Thus, utilizing the SMN protein as a biomarker useful for SMA requires further sensitivity enough to detect a difference in the level of the SMN protein expressed when a carrier is compared with a patient.

A quantitative analysis was actually attempted on the SMN protein by an ELISA method using peripheral blood mononuclear cells (Non-Patent Document 1). However, Kobayashi et al. reported that the result obtained by the ELISA method showed no significant difference in the level of the SMN protein expressed between childhood SMA patients and carriers. Further, Kobayashi et al. even disclosed that the level of the SMN protein expressed does not serve as a reliable indicator in the SMA diagnosis.

Moreover, in the SMN protein quantitative analysis by the ELISA method, peripheral blood mononuclear cells (PBMCs) have to be used, and operations such as centrifuging blood samples obtained from patients and others are burdensome, and also reduce the cell yield. Hence, the SMN protein quantification by the ELISA method using PBMCs also requires a large amount of blood to be collected.

In such circumstances, the inventors found a method for detecting the expression of SMN protein and reported that SMN protein can be utilized as a biomarker useful for childhood SMA (Patent Document 1).

However, Patent Document 1 clarified neither any SMN protein subcellular localization nor any SMN protein nuclear body.

Citation List

Patent Literatures

Patent Literature 1: WO2015/152410

Non Patent Literatures

Non Patent Literature 1: Dione T. Kobayashi et al., Plos one, November 2012, Vol. 7, Issue 11, e50763, Evaluation of Peripheral Blood Mononuclear Cell Processing and Analysis for Survival Motor Neuron Protein

Summary of Invention

An object of the present invention is to provide a method capable of analyzing an SMN protein nuclear body, which is a more reliable biomarker, using blood cell samples.

The present inventors have conducted earnest studies and as a result, the inventors have newly found out that analysis of an SMN protein nuclear body with imaging flow cytometry as well as enhancement of sensitivity of detecting the level of the SMN protein expressed can be achieved by selecting a particular cell population among blood cells in which SMN protein expression is to be detected and detecting the SMN protein expression in the cell population.

One aspect of the present invention provides a method for analyzing the expression of a survival motor neuron (SMN) protein nuclear body, comprising the steps of:

labeling one or more surface antigen markers of blood-derived nucleated cells in a sample containing the nucleated cells with one or more label antibodies, wherein the one or more surface antigen markers comprise CD33;
labeling SMN protein in the nucleated cells with a first fluorescent dye;
labeling nuclei of the nucleated cells;
selecting one cell population containing monocytes from a plurality of cell populations in which nuclei and SMN protein in the nucleated cells have been labeled and which have been classified based on the one or more surface antigen markers labeled with the one or more label antibodies; and
analyzing the expression of an SMN protein nuclear body of the selected cell population by measuring a fluorescence intensity emitted by the first fluorescent dye, wherein
the step of selecting a cell population and the step of analyzing the expression of an SMN protein nuclear body are performed by imaging flow cytometry which uses an objective lens with a magnification of 40 times or more.

In one aspect of the present invention, in the method for analyzing the expression of an SMN protein nuclear body, the step of labeling SMN protein in the nucleated cells comprises labeling SMN protein with a first fluorescent dye, and the step of labeling nuclei of the nucleated cells comprises labeling nuclei with a second fluorescent dye.

In one aspect of the present invention, the method for analyzing the expression of an SMN protein nuclear body further comprises the step of subjecting the sample containing the blood-derived nucleated cells to erythrocyte hemolysis treatment.

In one aspect of the present invention, in the method for analyzing the expression of an SMN protein nuclear body,

the sample containing the blood-derived nucleated cells is one obtained from a subject,

the step of analyzing the expression of an SMN protein nuclear body comprises quantifying an SMN protein nuclear body, and

the method comprises the step of comparing a result obtained by the quantification with a control selected from the following (a) to (c):

(a) if the subject is an SMA patient, the control is a result obtained in the same manner as in the method for quantifying the SMN protein nuclear body using blood obtained from the subject except that blood obtained from a healthy person or a carrier is used.

(b) if the subject is a healthy person or a carrier, the control is a result obtained in the same manner as in the method for quantifying the SMN protein nuclear body using blood obtained from the subject except that blood obtained from an SMA patient is used.

(c) if the subject is a person to whom a drug or a drug candidate against SMA has been administered, the control is a result obtained in the same manner as in the method for quantifying the SMN protein nuclear body using blood obtained from the subject after the administration of the drug or the drug candidate against SMA except that blood obtained from the subject before said administration is used.

According to one aspect of the present invention, it is possible to analyze an SMN protein nuclear body using a blood cell sample, the SMN protein nuclear body being a more highly reliable biomarker.

In addition, according to one aspect of the present invention, it is possible to provide a method which makes it possible to detect a difference in expression of an SMN protein nuclear body between SMA patients and healthy persons or carriers.

Furthermore, according to one aspect of the present invention, it is possible to perform expression analysis of an SMN protein nuclear body with about 500 $\mu$L of blood, and it is possible to provide a useful method in case of using an SMN protein nuclear body as a biomarker in a test of a drug or a drug candidate against SMA, in particular when collecting blood from a child patient.

Brief Description of Drawings

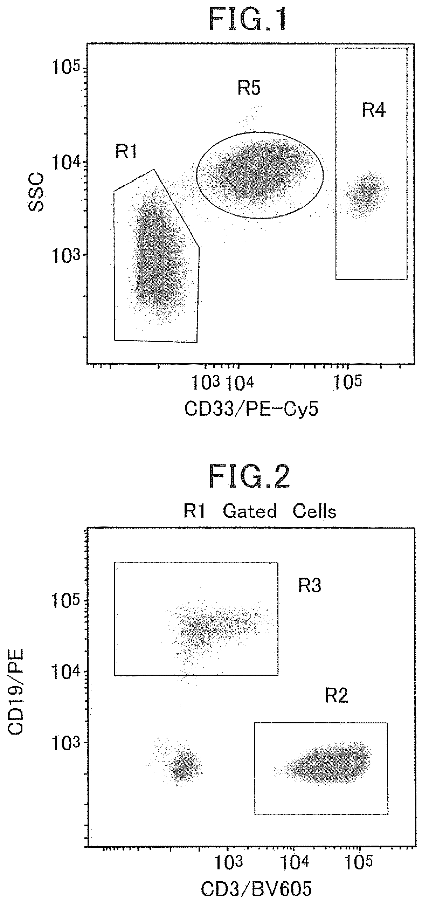

Fig. 1 illustrates a graph created by plotting fluorescence intensities measured by imaging flow cytometry using blood samples obtained from healthy persons. The vertical axis represents side scattering (SSC) and the horizontal axis represents the fluorescence intensity emitted by a fluorescent dye conjugated to the anti-CD33 antibody.

Fig. 2 is a further classification of a cell population, the cluster indicated by R1 in Fig. 1, and illustrates a graph created by plotting fluorescence intensities measured by imaging flow cytometry. The vertical axis represents the fluorescence intensity emitted by a fluorescent dye conjugated to the anti-CD19 antibody and the horizontal axis represents the fluorescence intensity emitted by a fluorescent dye conjugated to the anti-CD3 antibody.

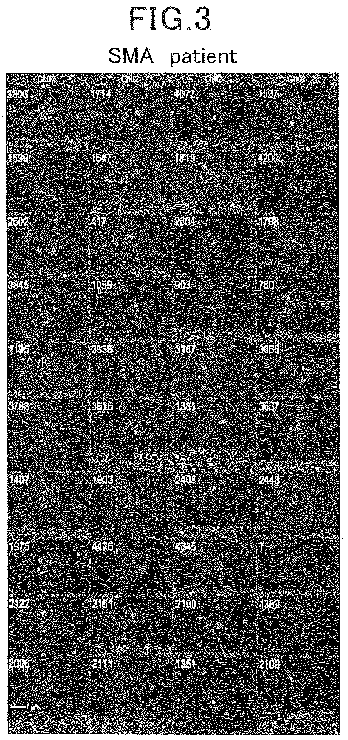

Fig. 3 is a cell photograph taken by imaging flow cytometry using a blood sample obtained from an SMA patient.

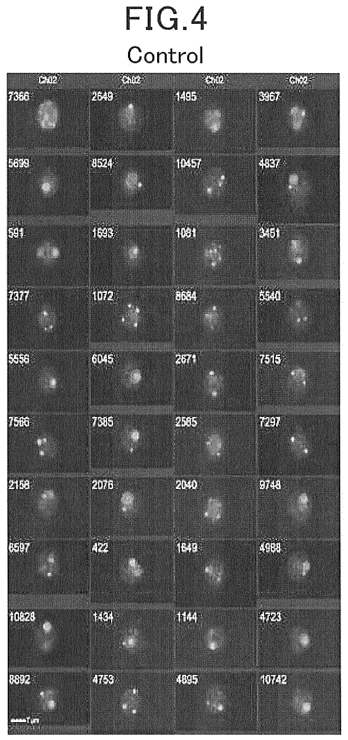

Fig. 4 is a cell photograph taken by imaging flow cytometry using a blood sample obtained from a healthy subject (control).

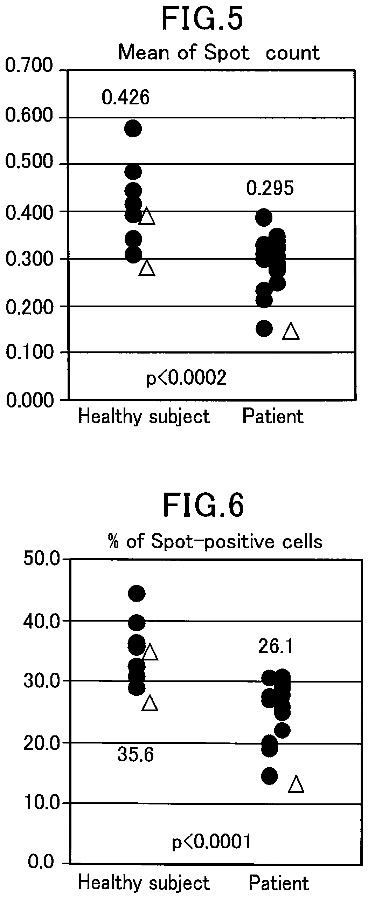

Fig. 5 is a graph illustrating the average value for the number of SMN protein nuclear bodies (spots) measured by imaging flow cytometry using respective blood samples obtained from healthy subjects and SMA patients. $\triangle$ represents the result obtained by using blood samples obtained from adults, and $\bigcirc$ represents the result obtained by using blood samples obtained from infants to children.

Fig. 6 is a graph illustrating the proportion of SMN protein nuclear body (spot) positive cells measured by imaging flow cytometry using respective blood samples obtained from healthy subjects and SMA patients. $\triangle$ represents the result obtained by using blood samples obtained from adults, and $\bigcirc$ represents the result obtained by using blood samples obtained from infants to children.

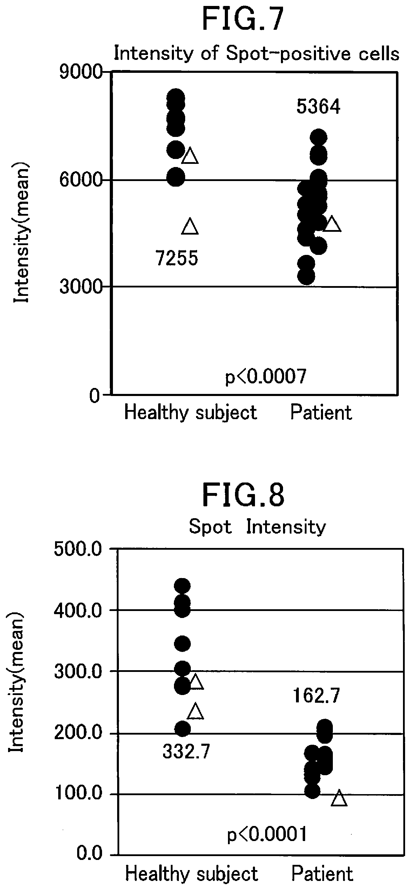

Fig. 7 is a graph illustrating the fluorescence intensity of SMN protein nuclear body (spot) positive cells measured by imaging flow cytometry using respective blood samples obtained from healthy subjects and SMA patients. $\triangle$ represents the result obtained by using blood samples obtained from adults, and $\bigcirc$ represents the result obtained by using blood samples obtained from infants to children.

Fig. 8 is a graph illustrating the fluorescence intensity of SMN protein nuclear bodies (spots) measured by imaging flow cytometry using respective blood samples obtained from healthy subjects and SMA patients. $\triangle$ represents the result obtained by using blood samples obtained from adults, and $\bigcirc$ represents the result obtained by using blood samples obtained from infants to children.

Description of Embodiments

Hereinafter, the present invention is described in detail.

A method for analyzing the expression of an SMN protein nuclear body of the present invention uses samples derived from blood obtained from subjects. The blood sample is less invasive to patients and is most suitable as a sample. For example, the subject is, but is not limited to, a childhood SMA patient, an adult SMA patient, a carrier, or a person not an SMA patient nor a carrier (a healthy person or a healthy subject). Childhood SMA patients include SMA type I patients, SMA type II patients and SMA type III patients.

A sample derived from blood obtained from a subject, used in the method of the present invention, may be any sample containing blood-derived nucleated cells. For a sample used in the method of the present invention, blood obtained from a subject, collected in a blood collection tube, may be used without any treatment, or a sample obtained from a subject may be treated in any way as long as the sample is treated to contain blood-derived nucleated cells. For example, a sample containing blood-derived nucleated cells used in the method of the present invention can include, but is not limited to, one obtained by centrifuging blood obtained from a subject and separating it as peripheral blood mononuclear cells (PBMC) and one containing nucleated cells obtained by hemolyzing red blood cells in blood and then centrifuging the resultant to precipitate it.

In the method of the present invention, technique conventionally known in the technical field of the present invention can be used to treat blood obtained from a subject so as to obtain a sample containing nucleated cells derived from the blood.

In the method of the present invention, the method for treating blood obtained from a subject to obtain a sample containing nucleated cells derived from the blood is preferably a method which results in a favorable cell yield with the least possible damage. For example, a method comprising hemolyzing red blood cells in blood, and then separating nucleated cells precipitated by centrifugation (lysis method) requires only a small amount of blood to be collected from a subject, and also imposes only a small stress to the blood cells. For these reasons, in the method of the present invention, the lysis method is preferably used to treat blood obtained from a subject so as to obtain a sample containing nucleated cells derived from the blood. Particularly, when SMN protein is utilized as a biomarker in testing a drug or a drug candidate against SMA, the lysis method is advantageously used because it requires only a small amount of blood to be collected from a child patient or the like.

Prior to the step of labeling one or more surface antigen markers of blood-derived nucleated cells in a sample containing the nucleated cells with one or more label antibodies, wherein the one or more surface antigen markers comprise CD33, blood obtained from a subject may be treated by using the lysis method or the lysis method may be used after the labeling step.

Also, in the method of the present invention, as a sample containing blood-derived nucleated cells, it is possible to use a sample containing cells subjected to lymphoblast genesis after separating blood-derived nucleated cells by conventionally known technique as described above. For the treatment of lymphoblast genesis, it is also possible to use technique conventionally known in the technical field of the present invention.

One embodiment of the method of the present invention comprises the step of preparing a sample containing blood-derived nucleated cells by centrifuging blood. Blood centrifugation can be performed at, for example, about 1400 to about 3200 rpm (190 to 1000 x g) for about 5 to about 20 minutes.

In the case where the step of preparing a sample containing blood-derived nucleated cells by centrifuging blood comprises separation of nucleated cells by the lysis method, centrifugation after hemolysis with a hemolytic agent is preferably performed at about 1400 to about 2300 rpm for about 5 to about 8 minutes. Likewise, in the case of using the lysis method after the step of labeling one or more surface antigen markers of nucleated cells with one or more label antibodies, centrifugation after hemolysis with a hemolytic agent is preferably performed at about 1400 to about 2300 rpm (190 to 510 x g) for about 5 to about 8 minutes.

In the case where the step of preparing a sample containing blood-derived nucleated cells by centrifuging blood comprises separation of PBMC, centrifugation is preferably performed at about 2000 to about 3200 rpm (390 to 1000 x g) for about 15 to about 20 minutes after collecting blood to a heparin tube and overlaying it with Ficoll solution or after placing blood in BD Vacutainer (registered trademark) CPT™ mononuclear cell separation blood collection tube.

It is possible to carry out the step of preparing a sample containing blood-derived nucleated cells by centrifuging blood using technique conventionally known in the technical field of the present invention.

The amount of blood derived from a subject used in the method of the present invention is not particularly limited as long as the amount enables expression analysis of an SMN protein nuclear body. The amount is, for example, about 0.5 mL or more and preferably about 1 mL or more, and about 3 mL or less and preferably about 2 mL or less. Note that conventional quantitative analysis of SMN protein by the ELISA method using peripheral blood mononuclear cells (PBMC) requires use of blood volume of at least 4 mL. Thus, one embodiment of the present invention is advantageous in that the amount of blood required is small.

As described above, a sample used in the method of the present invention shall contain nucleated cells derived from blood. However, since whole blood (peripheral blood) contains all sorts of nucleated cells, it can be said that whole blood contains a cell population rich in diversity and thus heterogeneous. Further, since human blood cell fractions constantly vary, it is preferable to minimize the influence of the variation in blood cell fractions on the amount of SMN protein expressed in blood.

In light of the above, the method for analyzing the expression of an SMN protein nuclear body of the present invention comprises the step of labeling one or more surface antigen markers of blood-derived nucleated cells in a sample containing the nucleated cells with one or more label antibodies, wherein the one or more surface antigen markers comprise CD33. By labeling surface antigen markers using label antigens as described above, nucleated cells in blood can be classified into several clusters (cell populations), for example, 2 to 4 clusters, and the SMN protein expression level can be measured for each cluster. In addition, the cell populations can be classified based on side scattering (SSC) together with the surface antigen markers. This makes it possible to reduce the influence of the variation in blood cell fractions on the amount of SMN protein expressed in blood.

Note that although the method for analyzing the expression of an SMN protein nuclear body of the present invention comprises the step of labeling SMN protein in nucleated cells and the step of labeling nuclei of nucleated cells, it is possible to perform in any order the three steps, namely the above two steps together with the step of labeling one or more surface antigen markers of blood-derived nucleated cells in a sample containing the nucleated cells with one or more label antibodies.

The surface antigen marker which is used in the method of the present invention is CD33. In the method of the present invention, surface antigen markers may be used alone, or together with two or more of other surface antigen markers including CD45, CD66, CD14, CD3, CD19, CD123, CD34, CD11c, CD25, HLA-DR. Among these surface antigen markers, CD45, CD66, CD3, CD19, and CD14 are generally known as a panhemocyte marker, a neutrophil marker, a T cell marker, a B cell marker, and a monocyte marker, respectively.

The method of the present invention comprises selection of cell populations containing monocytes. Cell populations containing monocytes can contain 95% or more, 90% or more, 85% or more, 80% or more, 75% or more, 70% or more, 65% or more, 60% or more, 55% or more, or 50% or more of monocytes. Since the method of the present invention comprises selecting cell populations containing monocytes, and performing, for those cell populations, expression analysis of an SMN protein nuclear body based on a label on SMN protein, significant difference can be observed in the fluorescence intensity of SMN protein nuclear body (spot) between healthy persons and SMA patients.

The method for analyzing the expression of an SMN protein nuclear body of the present invention comprises a step of labeling SMN protein in nucleated cells in a sample with a first fluorescent dye.

The SMN protein is a protein expressed by SMN gene which is a SMA responsible gene, and conceivably involved in motor neuron formation and so forth. The SMN gene includes the SMN1 gene and the SMN2 gene. Both are located in 5q13 on a long arm of chromosome 5. The SMN2 gene is different from the SMN1 gene by only one base in the coding region. However, assuming that the amount of full-length SMN1 mRNA transcribed from the SMN1 gene and to be translated into normal SMN protein is 100%, full-length SMN2 mRNA accounts for approximately 10% of the transcription products of the SMN2 gene, and truncated SMN2 mRNA in which deletion of exon 7 is observed accounts for approximately 90% of the transcription products of the SMN2 gene. The truncated SMN2 mRNA produces a non-functional protein, and has no use for the development of spinal motor neuron.

In the method of the present invention, technique conventionally known in the technical field of the present invention can be used to label SMN protein in a sample containing nucleated cells derived from blood. Herein, labeling the SMN protein should be able to label normal SMN protein translated from full-length SMN1 mRNA and full-length SMN2 mRNA, but may also label a non-functional protein translated from the truncated SMN2 mRNA.

Examples of the method for labeling the SMN protein include the use of an anti-SMN antibody conjugated to a labeling reagent; and the use of an anti-SMN antibody as a primary antibody not conjugated to a labeling reagent followed by the use of a secondary antibody conjugated to a labeling reagent. Examples of the labeling reagent include enzymes, dyes, fluorescent dyes, biotin, radioactive substances, various peptides, and amino acid linkers, but are not limited thereto. According to the present invention, the labeling reagent is a fluorescent dye, and examples thereof include fluorescein, rhodamine, coumarin, imidazole derivatives, indole derivatives, Cy3, Cy5, Cy5.5, Cy7, APC, PE, DyLight, AlexaFluor, and the like.

The anti-SMN antibody conjugated to a fluorescent dyes or the anti-SMN antibody used as a primary antibody, which is used in the method of the present invention, may be a monoclonal antibody or a polyclonal antibody, and commercially available products can be utilized. Examples of commercially available products of the anti-SMN antibodies include Milli-Mark™ of Millipore Corporation, antibodies provided by BD, Abcam plc., and Sigma-Aldrich Co., and other antibodies.

The method for analyzing the expression of an SMN protein nuclear body of the present invention comprises the step of labeling nuclei of nucleated cells in a sample. As a method for labeling nuclei, technique conventionally known in the technical field of the present invention can be used. For example, the method for labeling nuclei includes labeling with a fluorescent dye, labeling with an antibody recognizing cell nucleus-specific protein, and the like.

When using a fluorescent dye to label nuclei of blood-derived nucleated cells in a sample containing the nucleated cells in the method of the present invention, examples of the reagent include Hoechst 33342, Hoechst 33258, 4,6-diamino-2-phenylindole (DAPI), Propidium iodide (PI), fluorescence labeled anti-histone antibodies, fluorescence labeled anti-lamin antibodies, and the like.

In the present specification, a fluorescent dye for labeling SMN protein is referred to as a first fluorescent dye and a fluorescent dye for labeling nuclei is referred to as a second fluorescent dye in some cases.

In the method for analyzing the expression of an SMN protein nuclear body of the present invention, the step of labeling SMN protein in nucleated cells comprises labeling SMN protein with a first fluorescent dye, for example AlexaFluor 488, and the step of labeling nuclei of nucleated cells preferably comprises labeling nuclei with a second fluorescent dye, for example Hoechst 33342.

The method for analyzing the expression of an SMN protein nuclear body of the present invention comprises the step of selecting one cell population containing monocytes from a plurality of cell populations in which nuclei and SMN protein in nucleated cells have been labeled and which have been classified based on one or more surface antigen markers comprising CD33 labeled with one or more label antibodies. In one embodiment, the selecting step comprises selecting one cell population containing monocytes from a plurality of cell population in which the nuclei and the SMN protein in the nucleated cells have been labeled and which have been classified based on the one or more surface antigen markers comprising CD33 labeled with the one or more label antibodies and on side scattering (SSC).

Herein, the cell population containing monocytes in which nuclei and SMN protein have been labeled includes intact cells (viable cells) by 100%, 95% or more, 90% or more, 85% or more, 80% or more, 75% or more, 70% or more, 65% or more, 60% or more, 55% or more, or 50% or more of the population.

In one embodiment of the method of the present invention, the cell population of nucleated cells containing monocytes in which nuclei and SMN protein have been labeled is a population including cells having a nuclear fluorescence intensity of approximately $1 \times 10^5$ or more and a SMN protein fluorescence intensity of approximately $1 \times 10^3$ or more when the nuclei and the SMN protein are labeled with a fluorescent dye as described later in Example 1 of the present description and the fluorescence intensities are detected as described later in Example 1. Such cells are determined as intact cells. Additionally, cells keeping the cell form and also keeping the aspect ratio (width-to-height ratio) and surface area under microscope observation are determined as intact cells. The criteria of determining intact cells or determining a cell population including intact cells vary depending on the method for labeling nuclei and SMN protein in nucleated cells. However, the determinations can be made as appropriate based on the results of detecting nuclei and SMN protein in nucleated cells on the basis of a labeling reagent to be used.

The method for analyzing the expression of an SMN protein nuclear body of the present invention comprises the step of analyzing the expression of an SMN protein nuclear body of the cell population selected as described above based on a protein by measuring a fluorescence intensity emitted by the first fluorescent dye.

In the method of the present invention, the step of selecting a cell population containing monocytes and the step of analyzing the expression of an SMN protein nuclear body are performed by imaging flow cytometry which uses an objective lens with a magnification of 40 times or more. Such imaging flow cytometry makes it possible to perform expression analysis of an SMN protein nuclear body by focusing on the cell population mainly containing desired blood cell components (desired blood cell fractions) based on one or more surface antigen markers labeled with one or more label antibodies or on such surface antigen markers and side scattering (SSC). Therefore, the method of the present invention can be widely used in functional analysis of SMN protein, and can contribute greatly to diagnostic and therapeutic technique of SMA.

The magnification of an objective lens used is preferably 50 times or more and more preferably 60 times or more. Although the upper limit may be any value as long as it is within a range which enables expression analysis of an SMN protein nuclear body, the upper limit can be 200 times or less, 150 times or less, and 80 times or less.

Imaging flow cytometry can be carried out as appropriate according to the manufacturer's instructions using commercially available equipment.

Note that, in light of a fact that an SMN protein nuclear body was observed in the shape of a spot by the present invention, the present specification may simply refer to an SMN protein nuclear body observed in the shape of a spot as a "spot." The definition of the spot includes, for example, ones 1) existing in a nucleus, 2) having a size of 0.34 to 3.07 $\mu$m$^2$, 3) showing fluorescence intensity 5 times that of the background or greater, and 4) having a width-to-height ratio of 0.5 to 1.0. In addition, another definition of the spot includes, for example, ones 1) existing in a nucleus, 2) having a size of 0.34 to 8.54 $\mu$m$^2$, 3) showing fluorescence intensity 5.5 times that of the background or greater, and 4) having a width-to-height ratio of 0.4 to 1.0. In one embodiment of the method of the present invention, the SMN protein nuclear body of the selected cell population is observed in the shape of a spot, the spot being defined as existing in a nucleus, having a size of 0.34 to 8.54 $\mu$m$^2$, showing fluorescence intensity 5.5 times that of the background or greater, and having a width-to-height ratio of 0.4 to 1.0.

In the method for analyzing the expression of an SMN protein nuclear body of the present invention, the step of analyzing the expression of an SMN protein nuclear body by measuring a fluorescence intensity emitted by the first fluorescent dye may comprise quantifying the SMN protein nuclear body. In addition, the method of the present invention may comprise the step of comparing results of quantifying the SMN protein nuclear body between, for example, spinal muscular atrophy (SMA) patients, carriers, and healthy persons, specifically between SMA patients and carriers or between SMA patients and healthy persons. When comparison reveals a difference in results of quantifying the SMN protein (expression level or localization) between SMA patients and carriers or between SMA patients and healthy persons, it is possible to classify the SMA patients, the carriers, and the healthy persons.

In addition, the method for analyzing the expression of an SMN protein nuclear body of the present invention can be used in a method for screening a pharmaceutical agent for treating SMA.

In the method of the present invention, it is possible to appropriately perform an operation usually performed in detecting protein expression in cells, for example the step of washing cells with a reagent such as phosphate buffer solution.

Optionally, in the method for analyzing the expression of an SMN protein nuclear body of the present invention, a sample containing blood-derived nucleated cells is obtained from a subject, the step of analyzing the expression of an SMN protein nuclear body may comprise quantifying an SMN protein nuclear body, and the method may comprise the step of comparing results obtained by the quantification with control. Here, the control can be selected from the following (a) to (c):

(a) if the subject is an SMA patient, the control is a result obtained in the same manner as in the method for quantifying the SMN protein nuclear body using blood obtained from the subject except that blood obtained from a healthy person or a carrier is used;

(b) if the subject is a healthy person or a carrier, the control is a result obtained in the same manner as in the method for quantifying the SMN protein nuclear body using blood obtained from the subject except that blood obtained from an SMA patient is used; and

(c) if the subject is a person to whom a drug or a drug candidate against SMA has been administered, the control is a result obtained in the same manner as in the method for quantifying the SMN protein nuclear body using blood obtained from the subject after the administration of the drug or the drug candidate against SMA except that blood obtained from the subject before said administration is used.

Here, "measured in the same manner as" is construed such that, when comparing results of quantifying an SMN protein nuclear body (expression level or localization), changes to the extent, not substantially affecting the quantification of an SMN protein nuclear body, are permitted. For example, the meaning of the phrase does not require that steps such as washing cells be also the same.

Quantification of an SMN protein nuclear body can be carried out as appropriate by using technique conventionally known in the technical field of the present invention, such as a calibration curve method.

Here, it can be said that one embodiment of the present invention relates to a method for diagnosing SMA if it comprises the step of comparison as described above and the step of examining the subject based on the results of comparison. Moreover, it can also be said that one embodiment of the present invention relates to a method for screening a drug or a drug candidate against SMA if it comprises the step of comparison as described above and a step of selecting a drug or a drug candidate against SMA based on results of comparison.

Examples

Examples below provide a more detailed description of the present invention. Example 1: Expression Analysis of SMN Protein Nuclear Body

1. Preparation of Samples

Samples were prepared according to the following staining protocol. Note that all procedures were performed at room temperature unless otherwise noted.

(1) To a 15 mL conical tube, 1.5 mL of peripheral blood obtained from a subject was transferred.

(2) To the peripheral blood in the conical tube, 30 μL of Fc receptor blocking reagent (Clear Back, MTG-001, MBL) was added, followed by incubation for 15 minutes in the dark.

(3) To the peripheral blood in the conical tube after incubation, 5 μL of monoclonal antibodies against surface antigens (CD19, CD33, and CD3) (available from BioLegend) were added according to the following Tables 1 and 2, followed by incubation for 30 minutes in the dark.

[Table 1]

| | Ch1 | Ch2 | Ch3 | Ch4 | Ch5 | Ch6 |
|---|---|---|---|---|---|---|
| | Bright Field | | | | | ssc |
| Fluorescent Dye | | AF488 | R-PE | | PE-Cy5 | |
| Monoclonal Antibody | | SMN | CD19 | | CD33 | |
| Staining Pattern (Clone) | | | | | | |
| Stained | | 2B1 | HIB19 | | WM53 | |
| Isotype Ctrl. | | MOPC21 | HIB19 | | WM53 | |

[Table 2]

| | Ch7 | Ch8 | Ch9 | Ch10 | Ch11 | Ch12 |
|---|---|---|---|---|---|---|
| Fluorescent Dye | Hoechst | Bright | Field | BV610 | AF647 | AF750 |
| Monoclonal Antibody | Nucleus | | | CD3 | Gemin3 | ZNF259 |
| Staining Pattern (Clone) | | | | | | |
| Stained | Hoechst | | | UCHT1 | EPR11283 | EPR7595 |
| Isotype Ctrl. | Hoechst | | | UCHT1 | Rabbit IgG | Rabbit IgG |

AF: AlexaFluor

R-PE: R-phycoerythrin

PE-Cy5: R-phycoerythrin-cyanine 5

Hoechst: Hoechst 33342

BV: Brilliant Violet

(4) To each tube, 10 mL of lysing/fix solution (BD Phosflow™ Lyse/Fix Buffer) warmed in a 37°C water bath was added (well mixed, upside down 8 to 10 times).

(5) Incubation was performed for 10 minutes in a 37°C water bath.

(6) Centrifugation was performed at 300 x g for 5 minutes.

(7) The supernatant was removed until < 50 μL.

(8) The cells were resuspended with 15 mL of PBS(-), followed by centrifugation in the same manner as the above (6) and removal of the supernatant.

(9) The cells were resuspended with 1 mL of saponin contained permeabilization reagent (BD Phosflow™; 557885 (also referred to as BD Phosflow™ Perm/Wash Buffer I)), followed by incubation for 20 minutes in the dark.

(10) Centrifugation was performed in the same manner as the above (6).

(11) The cells were washed with 2 mL of permeabilization reagent (BD Phosflow™; 557885).

(12) The cells were resuspended with approximately 100 μL of permeabilization reagent (BD Phosflow™; 557885).

(13) In a 1.5 mL microtube, 95 μL of PBS(-) was prepared and 5 μL of cell suspension prepared in (12) above was added thereto, followed by 20-fold dilution to make a tube for staining. Likewise, it was diluted 20-fold to prepare a tube for control. In each tube, the concentration of cell suspension was $1 \times 10^6$ cells/about 50 μL.

(14-1) For the tube for staining, staining was performed with 1 μL of AF488-conjugated anti-human SMN monoclonal antibody (2B1), AF647-conjugated anti-human Gemin3 (EPR11283), and AF750-conjugated anti-human ZNF259 (EPR7595).

(14-2) For the tube for control, staining was performed with AF488-conjugated isotype control, AF647-conjugated isotype control, and AF750-conjugated isotype control in parallel with the above (14-1).

(15) Incubation was performed for 45 minutes in the dark.

(16) 500 μL of permeabilization reagent (BD Phosflow™; 557885) was added, followed by centrifugation at 300 × g for 5 minutes.

(17) The cells were resuspended with 500 μL of permeabilization reagent (BD Phosflow™; 557885), followed by centrifugation in the same manner as the above (16).

(18) The cells were resuspended with 50 μL of diluted Hoechst 33342 (0.25 μg/mL in PBS) prepared by diluting Hoechst 33342 (molecular probe (registered trademark)).

(19) Incubation was performed for 15 minutes in the dark.

(20) 500 μL of PBS(-) was added, followed by centrifugation in the same manner as the above (16).

(21) The cells were resuspended with 90 μL of PBS(-), followed by division into 3 tubes so that each of the tubes contained 30 μL of cell suspension.

## 2. Imaging Flow Cytometry

For the samples obtained as described above, expression analysis of an SMN protein nuclear body was performed by imaging flow cytometry (ImageStream (registered trademark) X Mark II Imaging Flow Cytometer, Amnis). The magnification used of the objective lens was 60 times, and a total of 50000 or more cells were subjected to imaging flow cytometry.

Note that among the samples obtained as described above, ones obtained from the following subjects were used.

Healthy persons (healthy subjects): 2 adult women (38 to 51 years old) and 8 children (1 to 7 years old) including men and women.

SMA patients: 1 adult man (21 years old) and 19 infants to children including men and women (2 months of age to 8 years of age)

### (1) Fractionation of Cell Populations

(i) From the image data including the whole fraction, the cell group with the correct focal length was selected using the focus parameter in the bright field.

(ii) Development was performed with cell size based on forward scattered light (FSC) and the width-to-height ratio of cell diameter, so that aggregated cells were excluded.

(iii) Nucleated cells were selected using the fluorescence intensity of nuclear DNA labeled by Hoechst 33342 as an index (exclusion of debris, dust, red blood cells, etc.).

(iv) Development was performed with internal structures of cells based on side scattered light (SSC) and cell surface antigen CD33 expression, so that the nucleated cells were divided into 3 groups. Fig. 1 shows the results. In Fig. 1, R1 represents a cell population mainly containing lymphocytes, R4 represents a cell population mainly containing monocytes, and R5 represents a cell population mainly containing neutrophils. Note that although Fig. 1 is a graph created by plotting the fluorescence intensities measured by imaging flow cytometry using blood samples obtained from healthy persons, the nucleated cells were also divided into three groups in the case of measurement using blood samples obtained from patients.

(v) The cell population mainly containing lymphocytes R1 was developed by expressions of cell surface antigens CD3 and CD19, so that it was divided into two groups, namely a cell population mainly containing CD3 positive T cells and a cell population mainly containing CD19 positive B cells. Fig. 2 shows the results. In Fig. 2, R2 represents a cell population mainly containing CD3 positive T cells and R3 represents a cell population mainly containing CD19 positive B cells.

The above analysis fractionated nucleated cells in peripheral blood into four groups. Note that this also applied to the case of measurement using blood sample obtained from a patient. In addition, although Fig. 2 shows the results obtained from one sample, nucleated cells in peripheral blood in each other sample were finally divided into the following four groups, in the same manner as the one sample.

R2: cell group mainly containing CD3 positive T cells ($SSC^{low}$, $CD33^-$, $CD3^+$)

R3: cell group mainly containing CD19 positive B cells ($SSC^{low}$, $CD33^-$, $CD19^+$)

R4: cell group mainly containing monocytes ($SSC^{intermediate}$, $CD33^{++}$)

R5: cell group mainly containing neutrophils ($SSC^{intermediate}$, $CD33^{intermediate}$)

### (2) Analysis of SMN Protein Nuclear Body (Spot) in Cell Group Mainly Containing Monocytes

Figs. 3 and 4 each illustrate a cell photograph taken by imaging flow cytometry in the same manner as described above. Figs. 3 and 4 are cell photographs of the cell groups mainly containing monocytes in the respective blood samples obtained from an SMA patient and a healthy subject (control). In Figs. 3 and 4, the SMN protein is indicated by green fluorescence. As is obvious from these cell photographs, it was observed that a healthy subject (control) was greater than an SMA patient in all of the fluorescence intensity in the whole cell, the fluorescence intensity in the nuclei, the number of spots, and the fluorescence intensity in the spots.

Note that the "spot" mentioned here means an SMN protein nuclear body observed in the shape of a spot, and the present examples employed the definition of the "spot" as one 1) existing in a nucleus, 2) having a size of 0.34 to 3.07 $\mu m^2$, 3) showing fluorescence intensity 5 times that of the background or greater, and 4) having a width-to-height ratio of 0.5 to 1.0.

(i) Average Value for Number of Spots

The average value of the number of spots in a cell group exhibiting a predetermined fluorescence intensity was calculated as follows.

(Aiming Adjustment in Nucleus)

In order to analyze the inside of the nucleus more accurately, the following operations were carried out.

1) Development was carried out with parameters of focus and contrast in the channel for detecting Hoechst 33342 which stained nucleus, followed by limitation to the cell group in the peak part of both parameters.
2) From the above cell group, 1000 or more cells showing the same degree of fluorescence intensity were selected as a cell group, using the fluorescence intensity of Hoechst 33342 as an index. This was set as the number of aiming adjusted cells.

(Subtraction of Isotype Control-Derived Data)

Evaluation was carried out using an analysis value which was obtained by subtracting the numerical value of a sample stained with a non-specific antibody (isotype control) from the numerical value of a sample stained with a specific antibody (anti-SMN antibody).

(Calculation of Average Value for Number of Spots)

The Mean was calculated using the number of spots/the number of aiming adjusted cells, and the average value of the number of spots was calculated with the following formula. Fig. 5 shows the results. In the graph of Fig. 5, the numerical values of 0.426 and 0.295 are average values (arithmetic means) of the number of spots calculated for all tested blood samples derived from healthy persons and blood samples derived from SMA patients, respectively.

[Formula 1]

$$\Delta \text{Mean} = \text{Mean}_{\text{fluorescence label-anti SMN monoclonal antibody}} - \text{Mean}_{\text{fluorescence label-isotype control}}$$

(ii) Proportion of Spot Positive Celes

The proportion of spot positive cells in a cell group exhibiting a predetermined fluorescence intensity was calculated as follows.

(Aiming Adjustment in Nucleus)

In order to analyze the inside of the nucleus more accurately, the following operations were carried out.

1) Development was carried out with parameters of focus and contrast in the channel for detecting Hoechst 33342 which stained nucleus, followed by limitation to the cell group in the peak part of both parameters.
2) From the above cell group, 1000 or more cells showing the same degree of fluorescence intensity were selected as a cell group, using the fluorescence intensity of Hoechst 33342 as an index. This was set as the number of aiming adjusted cells.

(Subtraction of Isotype Control-Derived Data)

Evaluation was carried out using an analysis value which was obtained by subtracting the numerical value of a sample stained with a non-specific antibody (isotype control) from the numerical value of a sample stained with a specific antibody (anti-SMN antibody).

(Calculation of Proportion of Spot Positive Cells)

The proportion (%) was calculated using the number of spots/the number of aiming adjusted cells × 100, and the proportion of spot positive cells was calculated with the following formula. Fig. 6 shows the results. In the graph of Fig. 6, the numerical values of 35.6 and 26.1 are average values (arithmetic means) of the proportion of spot positive cells calculated for all tested blood samples derived from healthy persons and blood samples derived from SMA patients, respectively.

[Formula 2]

$$\Delta\% = \%_{\text{fluorescence label-anti SMN monoclonal antibody}} - \%_{\text{fluorescence label-isotype control}}$$

(iii) Fluorescence Intensity of Spot Positive Cells

Expression of SMN protein limited to spot positive cells was analyzed.
Since the non-specific spot detection in samples stained with non-specific antibodies (isotype control) was suppressed to a small number (< 200 cells), it was impossible to calculate the fluorescence intensity of spot positive cells in samples stained with non-specific antibodies (isotype control). Thus, background subtraction by isotype control was considered unnecessary, and data calculated by specific antibodies (anti-SMN antibodies) was used for evaluation. Fig. 7 shows the results. In the graph of Fig. 7, the numerical values of 7255 and 5364 are average values (arithmetic means) of the fluorescence intensity of spot positive cells calculated for all tested blood samples derived from healthy persons and blood samples derived from SMA patients, respectively.

(iv) Fluorescence Intensity of Spots

Analysis was performed on the fluorescence intensity of spots themselves, that is, the expression of SMN protein nuclear body.
Since the non-specific spot detection in samples stained with non-specific antibodies (isotype control) was suppressed to a small number (<200 cells), it was impossible to calculate the fluorescence intensity of spots in spot positive cells in samples stained with non-specific antibodies (isotype control). Thus, background subtraction by isotype control was considered unnecessary, and data calculated by specific antibodies (anti-SMN antibodies) was used for evaluation. Fig. 8 shows the results. In the graph of Fig. 8, the numerical values of 332.7 and 162.7 are average values (arithmetic means) of the fluorescence intensity of spots calculated for all tested blood samples derived from healthy persons and blood samples derived from SMA patients, respectively.
From the above results, significant differences were observed at the significance level of 0.01 for normal human blood cells and blood cells derived from SMA patients in any of the average value of the number of spots, the proportion of spot positive cells, the fluorescence intensity of spot positive cells, and the fluorescence intensity of spots. Moreover, when comparing the results of the fluorescence intensity of spot positive cells with those of the fluorescence intensity of spots, the results of the fluorescence intensity of spot positive cells show that the relative ratio of normal human blood cells/blood cells derived from SMA patients is 1.35 (7255/5364), while the results of the fluorescence intensity of spots show the relative ratio of normal human blood cells/blood cells derived from SMA patients is 2.04. Therefore, it is suggested that it is possible to highly enhance the detection sensitivity based on results of the fluorescence intensity of spots. Specifically, the method for analyzing the expression of an SMN protein nuclear body of the present invention not only can simply detect the expression of SMN protein nuclear body, which is a more reliable biomarker, but also can measure the difference between healthy persons and SMA patients with higher detection sensitivity.

Industrial Applicability

SMA is one of the intractable diseases designated by the country, and the method for completely curing SMA has not been established yet. The present invention is extremely useful for the evaluation of methods which can be a treatment for SMA. For example, it is possible to use the method of the present invention in evaluating a drug or a drug candidate against SMA and in screening a drug or a drug candidate against SMA.
The present invention is also useful for use in the diagnosis of SMA. Furthermore, the present invention can be widely used in functional analysis of SMN protein, and can contribute greatly to diagnostic and therapeutic technique of SMA.

Therefore, it is expected that the present invention will make remarkable progress in the diagnostic and therapeutic technique of SMA, an intractable disease.

Still more, the present invention can be applied not only to SMA but also to technique for diagnosing or ameliorating diseases and symptoms caused by reduction in the expression level of SMN protein.

**Claims**

1. A method for analyzing the expression of a survival motor neuron (SMN) protein nuclear body, comprising the steps of:

   labeling one or more surface antigen markers of blood-derived nucleated cells in a sample containing the nucleated cells with one or more label antibodies, wherein the one or more surface antigen markers comprise CD33;
   labeling SMN protein in the nucleated cells with a first fluorescent dye;
   labeling nuclei of the nucleated cells;
   selecting one cell population containing monocytes from a plurality of cell populations in which the nuclei and the SMN protein in the nucleated cells have been labeled and which have been classified based on the one or more surface antigen markers comprising CD33 labeled with the one or more label antibodies; and
   analyzing the expression of an SMN protein nuclear body of the selected cell population by measuring a fluorescence intensity emitted by the first fluorescent dye, wherein
   the step of selecting a cell population and the step of analyzing the expression of an SMN protein nuclear body are performed by imaging flow cytometry which uses an objective lens with a magnification of 40 times or more.

2. The method according to claim 1, wherein
   the selecting step comprises selecting one cell population containing monocytes from a plurality of cell population in which the nuclei and the SMN protein in the nucleated cells have been labeled and which have been classified based on the one or more surface antigen markers comprising CD33 labeled with the one or more label antibodies and on side scattering (SSC).

3. The method according to claim 1 or 2, wherein

   the step of labeling SMN protein comprises labeling SMN protein with a first fluorescent dye, and
   the step of labeling nuclei comprises labeling nuclei with a second fluorescent dye.

4. The method according to any one of claims 1 to 3, further comprising the step of subjecting the sample containing the blood-derived nucleated cells to erythrocyte hemolysis treatment.

5. The method according to any one of claims 1 to 4, wherein

   the sample containing the blood-derived nucleated cells is one obtained from a subject,
   the step of analyzing the expression of an SMN protein nuclear body comprises quantifying an SMN protein nuclear body, and
   the method comprises the step of comparing a result obtained by the quantification with a control selected from the following (a) to (c):

      (a) if the subject is a spinal muscular atrophy (SMA) patient, the control is a result obtained in the same manner as in the method for quantifying the SMN protein nuclear body using blood obtained from the subject except that blood obtained from a healthy person or a carrier is used;
      (b) if the subject is a healthy person or a carrier, the control is a result obtained in the same manner as in the method for quantifying the SMN protein nuclear body using blood obtained from the subject except that blood obtained from an SMA patient is used; and
      (c) if the subject is a person to whom a drug or a drug candidate against SMA has been administered, the control is a result obtained in the same manner as in the method for quantifying the SMN protein nuclear body using blood obtained from the subject after the administration of the drug or the drug candidate against SMA except that blood obtained from the subject before said administration is used.

6. The method according to any one of claims 1 to 5, wherein the SMN protein nuclear body of the selected cell population is observed in the shape of a spot, the spot being defined as existing in a nucleus, having a size of 0.34 to 8.54 $\mu$m$^2$, showing fluorescence intensity 5.5 times that of the background or greater, and having a width-to-height ratio of 0.4 to 1.0.

**Patentansprüche**

1. Verfahren zum Analysieren der Expression eines Proteinnuklearkörpers eines Überlebensmotorneurons (SMN), umfassend die Schritte des:

Markierens von einem oder mehreren Oberflächenantigenmarkern von aus Blut stammenden nukleierten Zellen in einer Probe, welche die nukleierten Zellen enthält, mit einem oder mehreren Markierungsantikörpern, wobei der eine oder die mehreren Oberflächenantigenmarker CD33 umfassen,
Markierens eines SMN-Proteins in den nukleierten Zellen mit einem ersten fluoreszierenden Farbstoff,
Markierens von Nuklei der nukleierten Zellen,
Auswählens einer Zellpopulation, die Monozyten enthält, aus mehreren Zellpopulationen, in denen die Nuklei und das SMN-Protein in den nukleierten Zellen markiert worden sind und die basierend auf dem einen oder den mehreren Oberflächenantigenmarkern, die CD33 umfassen und die mit dem einen oder den mehreren Markierungsantikörpern markiert sind, klassifiziert worden sind, und
Analysierens der Expression eines SMN-Proteinnuklearkörpers der ausgewählten Zellpopulation durch Messen einer Fluoreszenzintensität, die durch den ersten fluoreszierenden Farbstoff emittiert wird, wobei
der Schritt des Auswählens einer Zellpopulation und der Schritt des Analysierens der Expression eines SMN-Proteinnuklearkörpers durch bildgebende Durchflusszytometrie durchgeführt werden, die eine Objektivlinse mit einer 40-fachen Vergrößerung oder mehr verwendet.

2. Verfahren nach Anspruch 1, wobei
der Auswahlschritt das Auswählen einer Zellpopulation umfasst, die Monozyten aus mehreren Zellpopulationen enthält, in denen die Nuklei und das SMN-Protein in den nukleierten Zellen markiert worden sind und die basierend auf dem einen oder den mehreren Oberflächenantigenmarkern, die CD33 umfassen und die mit dem einen oder den mehreren Markierungsantikörpern markiert sind, und auf Seitenstreuung (SSC) klassifiziert worden sind.

3. Verfahren nach Anspruch 1 oder 2, wobei

der Schritt des Markierens eines SMN-Proteins das Markieren eines SMN-Proteins mit einem ersten fluoreszierenden Farbstoff umfasst, und
der Schritt des Markierens von Nuklei das Markieren von Nuklei mit einem zweiten fluoreszierenden Farbstoff umfasst.

4. Verfahren nach einem der Ansprüche 1 bis 3, ferner umfassend den Schritt des Aussetzens der Probe, welche die aus Blut stammenden nukleierten Zellen enthält, gegenüber einer Erythrozytenhämolysebehandlung.

5. Verfahren nach einem der Ansprüche 1 bis 4, wobei

die Probe, welche die aus Blut stammenden nukleierten Zellen enthält, eine ist, die von einem Subjekt erhalten wird,
der Schritt des Analysierens der Expression eines SMN-Proteinnuklearkörpers das Quantifizieren eines SMN-Proteinnuklearkörpers umfasst, und
das Verfahren den Schritt des Vergleichens eines Ergebnisses, das durch die Quantifizierung erhalten wird, mit einer Kontrolle umfasst, die aus dem Folgenden (a) bis (c) ausgewählt ist:

(a) wenn das Subjekt ein Patient mit spinaler Muskelatrophie (SMA) ist, ist die Kontrolle ein Ergebnis, das auf die gleiche Weise wie in dem Verfahren zum Quantifizieren des SMN-Proteinnuklearkörpers unter Verwendung von Blut erhalten wird, das von dem Subjekt erhalten wird, außer dass Blut, das von einer gesunden Person oder einem gesunden Träger erhalten wird, verwendet wird,
(b) wenn das Subjekt eine gesunde Person oder ein gesunder Träger ist, ist die Kontrolle ein Ergebnis, das auf die gleiche Weise wie in dem Verfahren zum Quantifizieren des SMN-Proteinnuklearkörpers unter Verwendung von Blut erhalten wird, das von dem Subjekt erhalten wird, außer dass Blut, das von einem SMA-Patienten erhalten wird, verwendet wird, und

(c) wenn das Subjekt eine Person ist, der ein Arzneimittel oder ein Arzneimittelkandidat gegen SMA verabreicht worden ist, ist die Kontrolle ein Ergebnis, das auf die gleiche Weise wie in dem Verfahren zum Quantifizieren des SMN-Proteinnuklearkörpers unter Verwendung von Blut erhalten wird, das von dem Subjekt nach der Verabreichung des Arzneimittels oder des Arzneimittelkandidaten gegen SMA erhalten wird, außer dass Blut, das von dem Subjekt vor der Verabreichung erhalten wird, verwendet wird.

6. Verfahren nach einem der Ansprüche 1 bis 5, wobei der SMN-Proteinnuklearkörper der ausgewählten Zellpopulation in der Form eines Flecks beobachtet wird, wobei der Fleck als in einem Nukleus vorhanden, eine Größe von 0,34 bis 8,54 $\mu m^2$ aufweisend, eine 5,5-fache Fluoreszenzintensität gegenüber derjenigen des Hintergrunds oder mehr zeigend und ein Verhältnis von Breite zu Höhe von 0,4 zu 1,0 aufweisend definiert ist.

**Revendications**

1. Procédé pour l'analyse de l'expression d'un corps nucléaire de la protéine neurone moteur de survie (SMN), comprenant les étapes de :

   marquage d'un ou plusieurs marqueurs d'antigènes de surface de cellules nucléées dérivées du sang dans un échantillon contenant les cellules nucléées avec un ou plusieurs anticorps de marqueurs, dans lequel les un ou plusieurs marqueurs d'antigènes de surface comprennent CD33 ;
   marquage de la protéine SMN dans les cellules nucléées avec un premier colorant fluorescent ;
   marquage de noyaux des cellules nucléées ;
   sélection d'une population de cellules contenant des monocytes à partir de plusieurs populations de cellules dans lesquelles les noyaux et la protéine SMN dans les cellules nucléées ont été marqués et qui ont été classés sur la base des un ou plusieurs marqueurs d'antigènes de surface comprenant CD33 marqués avec les un ou plusieurs anticorps de marqueurs ; et
   analyse de l'expression d'un corps nucléaire de la protéine SMN de la population de cellules sélectionnée en mesurant une intensité de fluorescence émise par le premier colorant fluorescent, dans lequel
   l'étape de sélection d'une population de cellules et l'étape d'analyse de l'expression d'un corps nucléaire de la protéine SMN sont réalisées par imagerie en cytométrie de flux qui utilise une lentille d'objectif avec un agrandissement de 40 fois ou plus.

2. Procédé selon la revendication 1, dans lequel
   l'étape de sélection comprend la sélection d'une population de cellules contenant des monocytes à partir de plusieurs populations de cellules dans lesquelles les noyaux et la protéine SMN dans les cellules nucléées ont été marqués et qui ont été classés sur la base des un ou plusieurs marqueurs d'antigènes de surface comprenant CD33 marqués avec les un ou plusieurs anticorps de marqueurs et sur une dispersion latérale (SSC).

3. Procédé selon la revendication 1 ou 2, dans lequel

   l'étape de marquage de la protéine SMN comprend le marquage de la protéine SMN avec un premier colorant fluorescent, et
   l'étape de marquage de noyaux comprend le marquage de noyaux avec un second colorant fluorescent.

4. Procédé selon l'une quelconque des revendications 1 à 3, comprenant de plus l'étape soumettant l'échantillon contenant les cellules nucléées dérivées du sang à un traitement d'hémolyse d'érythrocyte.

5. Procédé selon l'une quelconque des revendications 1 à 4, dans lequel

   l'échantillon contenant les cellules nucléées dérivées du sang est un obtenu chez un sujet,
   l'étape d'analyse de l'expression d'un corps nucléaire de la protéine SMN comprend la quantification d'un corps nucléaire de la protéine SMN, et
   le procédé comprend l'étape de comparaison d'un résultat obtenu par la quantification avec un témoin choisi parmi les (a) à (c) suivants :

   (a) si le sujet est un patient ayant une amyotrophie spinale (SMA), le témoin est un résultat obtenu de la même manière que dans le procédé pour la quantification du corps nucléaire de la protéine SMN utilisant du sang obtenu chez le sujet à l'exception que l'on utilise du sang obtenu chez une personne saine ou un porteur ;

(b) si le sujet est une personne saine ou un porteur, le témoin est un résultat obtenu de la même manière que dans le procédé pour la quantification du corps nucléaire de la protéine SMN utilisant du sang obtenu chez le sujet à l'exception que l'on utilise du sang obtenu chez un patient de SMA ; et

(c) si le sujet est une personne à laquelle un médicament ou un médicament candidat contre SMA a été administré, le témoin est un résultat obtenu de la même manière que dans le procédé pour la quantification du corps nucléaire de la protéine SMN utilisant du sang obtenu chez le sujet après l'administration du médicament ou du médicament candidat contre SMA à l'exception que l'on utilise du sang obtenu chez le sujet avant ladite administration.

6. Procédé selon l'une quelconque des revendications 1 à 5, dans lequel le corps nucléaire de la protéine SMN de la population de cellules sélectionnée est observé dans la forme d'une tache, la tache étant définie comme existant dans un noyau, présentant une dimension de 0,34 à 8,54 $\mu$m$^2$, présentant une intensité de fluorescence 5,5 fois celle du bruit de fond ou supérieure, et présentant un rapport largeur- hauteur de 0,4 à 1,0.

# FIG.1

# FIG.2

### R1 Gated Cells

# FIG.3

## SMA  patient

# FIG.4

## Control

# FIG.5

Mean of Spot count

p<0.0002

Healthy subject    Patient

# FIG.6

% of Spot-positive cells

p<0.0001

Healthy subject    Patient

# FIG.7

Intensity of Spot-positive cells

# FIG.8

Spot Intensity

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015152410 A

**Non-patent literature cited in the description**

- **DIONE T. KOBAYASHI et al.** Evaluation of Peripheral Blood Mononuclear Cell Processing and Analysis for Survival Motor Neuron Protein. *Plos one,* November 2012, vol. 7 (11), e50763